# EUROPEAN PATENT APPLICATION

(11) **EP 3 424 407 A1**
(43) Date of publication of application: **09.01.2019**
(21) Application number: 17184471.5
(22) Date of filing: 02.08.2017
(51) Int. Cl.: A61B 5/00

(54) **PHYSIOLOGICAL MONITORING DEVICES AND PHYSIOLOGICAL MONITORING METHOD**

(30) Priority: 03.07.2017 US 201715640662
(71) Applicant: MediaTek Inc., Hsin-Chu 300 (TW)
(72) Inventor: CHEN, Tsan-Jieh, 300 Hsinchu City (TW); HSU, Shu-Yu, 114 Taipei City (TW)
(74) Representative: Hoefer & Partner Patentanwälte mbB

(57) **Abstract**

A physiological monitoring device is provided. The physiological monitoring device includes a physiological sensor, a quality estimator, and a feature extractor. The physiological sensor is configured to sense a physiological feature to generate a bio-signal. The quality estimator estimates quality of the bio-signal. The feature extractor receives the bio-signal and performs a predetermined operation to the bio-signal to obtain feature data. The amount of computation induced by the predetermined operation for the feature extractor is changed according to the estimated quality of the bio-signal.

## Description

### BACKGROUND OF THE INVENTION

### Field of the Invention

The invention relates to a physiological monitoring device, and more particularly to a physiological monitoring device which consumes less power during the monitoring period.

### Description of the Related Art

Wearable devices are a hot topic these years, Some wearable devices are capable of tracking medically health information, such as electrocardiography (ECG), photoplethysmogram (PPG), heart rate, and blood pressure. In order to obtain the change of the health information for diagnosis of diseases, these wearable devices enable continuous healthcare monitoring. However, the continuous healthcare monitoring increases power consumption. In order to reduce the power consumption, these wearable devices may lower sampling rates for bio-signals or lower computation rates of their processors, which limits the capability of the these wearable devices and degrades the accuracy of the healthcare monitoring.

### BRIEF SUMMARY OF THE INVENTION

An exemplary embodiment of a physiological monitoring device. The physiological monitoring device comprises a physiological sensor, a quality estimator, and a feature extractor. The physiological sensor is configured to sense a physiological feature to generate a bio-signal. The quality estimator estimates quality of the bio-signal. The feature extractor receives the bio-signal and performs a predetermined operation to the bio-signal to obtain feature data. The amount of computation induced by the predetermined operation for the feature extractor is changed according to the estimated quality of the bio-signal.

An exemplary embodiment of a physiological monitoring method is provided. The physiological monitoring method comprises the steps of sensing a physiological feature by a physiological sensor; estimating quality of the bio-signal; performing a predetermined operation to the bio-signal to obtain feature data by a feature extractor; and changing an amount of computation induced by the predetermined operation for the feature extractor according to the estimated quality of the bio-signal.

A detailed description is given in the following embodiments with reference to the accompanying drawings.

### BRIEF DESCRIPTION OF THE DRAWINGS

The invention can be more fully understood by reading the subsequent detailed description and examples with references made to the accompanying drawings, wherein:
FIG. 1 shows one exemplary embodiment of a physiological monitoring device;
FIG. 2A shows a bio-signal sensed by the physiological monitoring device of FIG. 1;
FIG. 2B is a schematic diagram showing that a feature extractor of the physiological monitoring device of FIG. 1 stops performing a predetermined operation during some periods according to a determined result related to a motion signal;
FIG. 3 is a schematic diagram showing that a feature extractor of the physiological monitoring device of FIG. 1 stops performing a predetermined operation during some periods according to a determined result related to a contact impedance;
FIG. 4 shows an exemplary photoplethysmogram (PPG) signal with low quality;
FIG. 5 is a schematic diagram showing that a feature extractor of the physiological monitoring device of FIG. 1 stops performing a predetermined operation during some periods according to a determined result related to a correction rate;
FIG. 6 shows another exemplary embodiment of a physiological monitoring device;
FIG. 7 is a schematic diagram showing that a bio-sensor of the physiological monitoring device of FIG. 6 is not powered by a supply voltage operation during some periods according to a determined result related to a motion signal;
FIG. 8 shows another exemplary embodiment of a physiological monitoring device; and
FIGs. 9-11 are schematic diagrams showing that some signals are not recorded into a memory device, thereby reducing power consumption.

### DETAILED DESCRIPTION OF THE INVENTION

The following description is of the best-contemplated mode of carrying out the invention. This description is made for the purpose of illustrating the general principles of the invention and should not be taken in a limiting sense. The scope of the invention is best determined by reference to the appended claims.

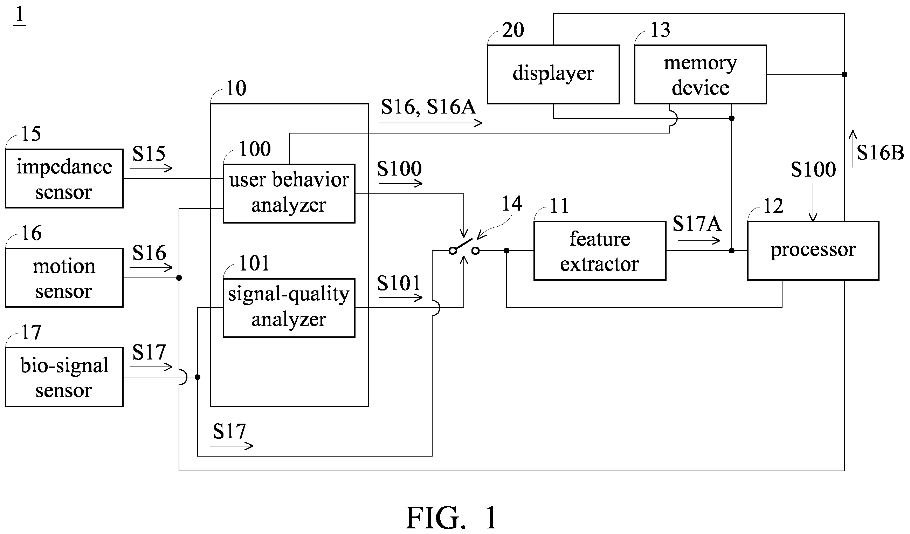

FIG. 1 shows one exemplary embodiment of a physiological monitoring device. As shown in FIG. 1, a physiological monitoring device 1 comprises a quality estimator 10, a feature extractor 11, an application processor 12, a memory device 13, a switch 14, an impedance sensor 15, a motion sensor 16, at least one physiological sensor 17, a power supplier 18, and a displayer 20. The physiological monitoring device 1 can be a wearable device with a healthcare function and/or a motion recoding function, such as a smart watch, or a physiological monitor, such as an ExG monitor used to monitor at least one of electrocardiography (ECG), electroencephalograph (EEG), electromyography (EMG), electrooculography (EOG), electroretinogram (ERG), electrogastrography (EGG), and electroneurogram (ENG) of the user, a photoplethysmogram (PPG) monitor, a heart rate monitor, or an oximeter. In the embodiment, one physiological sensor 17 is given as an example. However, in other embodiments, the number of physiological sensor 17 is determined by the system requirement. When the physiological sensor 17 is powered by the power supplier 18, the physiological sensor 17 works to sense a physiological feature of the user. According to the embodiment, the physiological sensor 17 can be implemented by electrodes, an infrared sensor, or a pressure cuff to sense a physiological feature of the user who is wearing, holding or contacting the physiological sensor 17, such as the ECG, EEG, EMG, EOG, ERG, EGG, ENG, PPG, heart beats, or oxygen saturation of the user. The physiological sensor 17 generates a bio-signal S17 according to the sensed result. In the embodiment, based on the sensed physiological feature, the bio-signal S17 is an ECG signal, an EGG signal, an EMG signal, an EOG signal, an ERG signal, an EGG signal, a PPG signal, or a heart-beat signal. The switch 14 is coupled between the bio-signal sensor 17 and the feature extractor 11. When the switch 14 is turned on, a transmission path from the bio-signal sensor 17 to the feature extractor 11 is provided. When the feature extractor 11 receives the bio-signal S 17 through the transmission path, the feature extractor 11 performs at least one predetermined operation to the bio-signal S17 to obtain a feature signal S17A carrying a plurality of recodes of feature data. The feature extractor 11 may transmits the obtained feature signal S 17A to the displayer 20, and the displayer 20 can show values, diagrams, or waveforms related to the feature signal S 17A which represent the physiological feature of the user. For example, a viewer, such as a doctor, may make a diagnosis of a disease according to the values, diagrams, or waveforms shown on the displayer 20. The feature extractor 11 may also transmits the obtained feature signal S17A to the processor 12 for advanced analysis.

The quality of the bio-signal S17 affects the accuracy of the feature data. For example, if the quality of the bio-signal S17 is low, the accuracy of the feature data is low, so that the values, diagrams, or waveforms shown on the displayer 20 cannot represent the real physiological feature of the user. How to estimate the quality of the bio-signal S17 will be described in the following paragraphs.

When the user is wearing, holding, or contacting the physiological sensor 17, the impedance sensor 15 is disposed close to the physiological sensor 17 to sense the contact impedance of the physiological sensor 17. The contact impedance indicates whether the user wears, holds, or contacts the physiological sensor 17 properly. When the user wears, holds, or contacts the physiological sensor 17 properly, the bio-sensor 17 can sense the physiological feature correctly, and the quality of the bio-signal S17 is high. Thus, the contact impedance can be one parameter for estimating the quality of the bio-signal S17. The impedance sensor 15 generates an impedance signal S15 according to the sensed contact impedance. In one embodiment, the impedance sensor 15 and the physiological sensor 17 are disposed on the same base. In another embodiment, the impedance sensor 15 is integrated with the physiological sensor 17 to form a sensor which can sense at least one physiological feature of the user and the contact impedance.

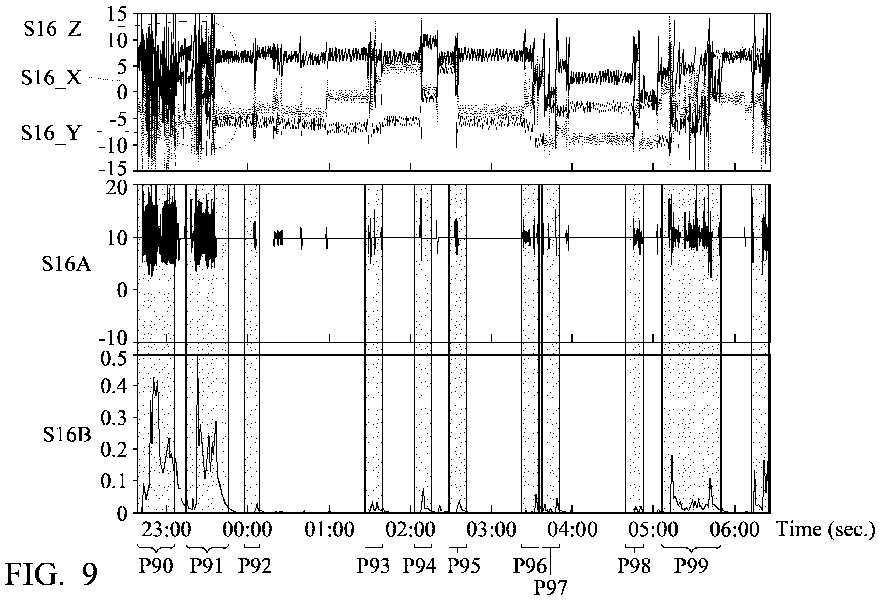

When the user is wearing, holding, or contacting the physiological sensor 17, the motion sensor 15 is disposed on a specific portion of the body of the user, such as one arm, one wrist, or one leg of the user, to sense the motion or activity of the user. The motion sensor 16 generates a motion signal S 15 according to the sensed motion or activity. For example, when the user is exercising or moving the limbs, the value of the motion signal S15 (that is the amplitude of the motion signal S15) is large. In cases in which the user is exercising or moving the limbs, the bio-sensor 17 cannot sense the physiological feature (such as ECG, EGG) correctly. Thus, the motion signal S15 can be one parameter for estimating the quality of the bio-signal S17. In an embodiment, the motion sensor 16 is implemented by an inertial sensor, such as a G-sensor, and the motion signal S16 comprises an X-direction component S16_X, a Y-direction component S16_Y, and a Z-direction component S16_Z (shown in FIG. 9).

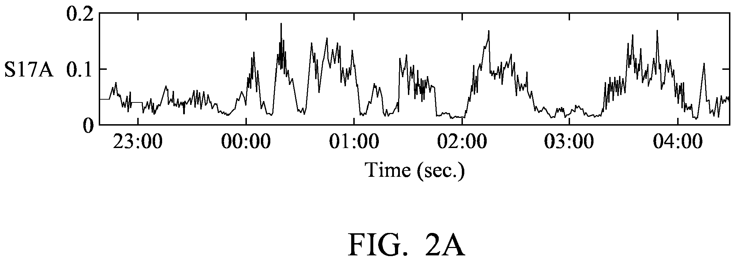

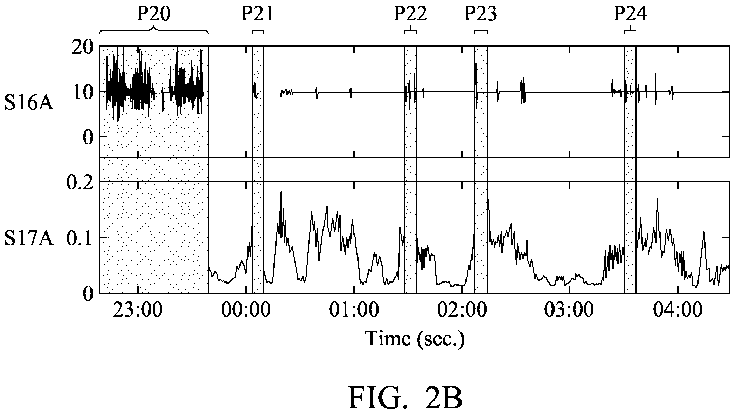

The quality estimator 10 operates to estimate the quality of the bio-signal S17; in other words, the quality estimator 10 operates to determine whether the quality of the bio-signal S17 is good enough to be a proper signal which will be processed by the feature extractor 11 to obtain the feature data. In the embodiment, referring to FIG. 1, the quality estimator 10 comprises a user behavior analyzer 100 and a signal-quality analyzer 101. The user behavior analyzer 100 receives the motion signal S16 and normalized the X-direction component S16X, the Y-direction component S16Y, and the Z-direction component S16Z of the motion signal S16. Referring to FIG. 2B, a signal S16A is shown to represent the normalized motion signal. The user behavior analyzer 100 determines whether the value of the normalized motion signal S16A (that is, the normalized value of the motion signal S16) is larger than a motion threshold. In an embodiment, when the user behavior analyzer 100 receives the motion signal S16, the user behavior analyzer 100 may record the motion signal S16 and the normalized motion signal S16A into the memory device 13. The user behavior analyzer 100 also receives the impedance signal S15. The user behavior analyzer 100 determines whether the value of the impedance signal S15 is larger than an impedance threshold. The user behavior analyzer 100 generates a control signal S100 according to the determined result related to the value of the normalized motion signal S16A and the determined result related to the value of the impedance signal S15.The control signal S100 is coupled to control the turned-on/off state of the switch 14. Referring to FIG. 2B, the user behavior analyzer 100 determines that the value of the normalized motion signal S16A is larger than the motion threshold during period P20∼P24, which indicates that the user is exercising or moving the limbs and that the quality of the bio-signal S 17 is low. The switch 14 is turned off according to the control signal S100 during the period P20∼P24. Thus, the bio-signal S17 is not provided to the feature extractor 11 during the period P20∼P20. As shown in FIG. 2B, since the feature extractor 11 does not receive any the bio-signal S 17 during the period P20∼P20, the feature extractor 11 does not perform the predetermined operation, so that no feature data is obtained during the period P20∼P20. Compared with the feature signal S 17A of FIG. 2A which is obtained by the feature extractor 11 through performing the predetermined operation all the time, the number of recodes of feature data in FIG. 2B is less, which indicates that the amount of computation induced by the predetermined operation for the feature extractor 11 is decreased. According to the embodiment, since the feature extractor 11 does not perform the predetermined operation all the time, the power consumption of the physiological monitoring device 1 is reduced.

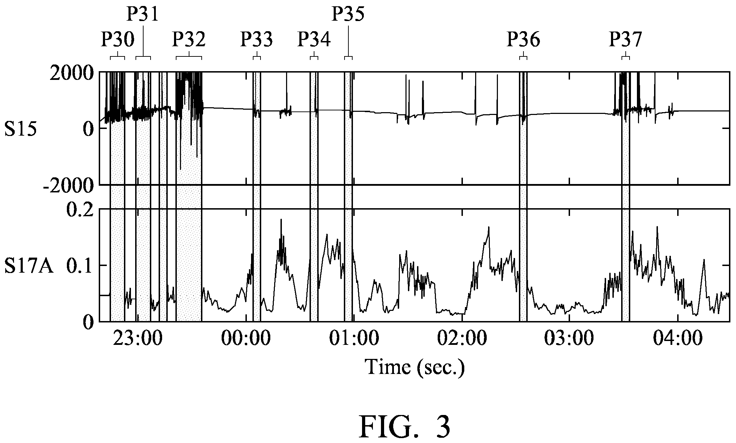

Referring to FIG. 3, the user behavior analyzer 100 determines that the value of the impedance signal S5A is larger than the motion threshold during period P30∼P37, which indicates that the user does not wear, hold, or contact the physiological sensor 17 properly and that the quality of the bio-signal S17 is low. The switch 14 is turned off according to the control signal S100 during the period P30∼P37. Thus, the bio-signal S17 is not provided to the feature extractor 11 during the period P30∼P37. As shown in FIG. 3, since the feature extractor 11 does not receive any the bio-signal S 17 during the period P30∼P37, the feature extractor 11 does not perform the predetermined operation, so that no feature data is obtained during the period P30∼P37. Compared with the feature signal S 17A of FIG. 2A which is obtained by the feature extractor 11 through performing the predetermined operation all the time, the number of recodes of feature data in FIG. 3 is less, which indicates that the amount of computation induced by the predetermined operation for the feature extractor 11 is decreased. According to the embodiment, since the feature extractor 11 does not perform the predetermined operation all the time, the power consumption of the physiological monitoring device 1 is reduced. According to the embodiment, once the value of the normalized motion signal S16A is larger than the motion threshold or once the value of the impedance signal S5A is larger than the motion threshold, the user behavior analyzer 100 generates the control signal S100 to turn off the switch 14, thereby decreasing the amount of computation induced by the predetermined operation.

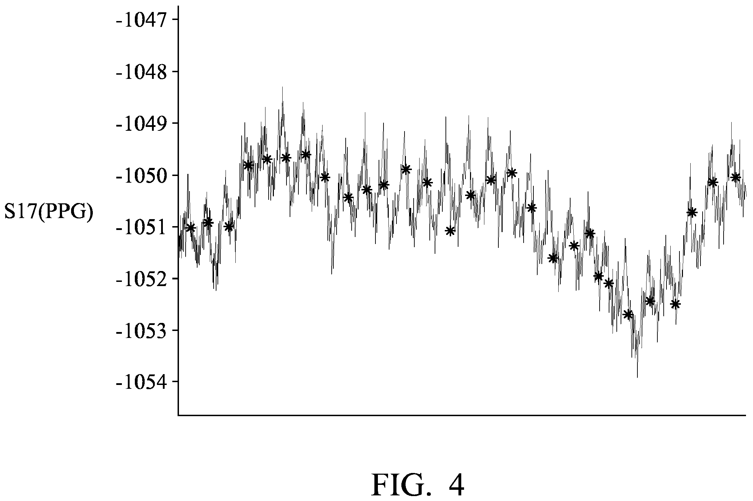

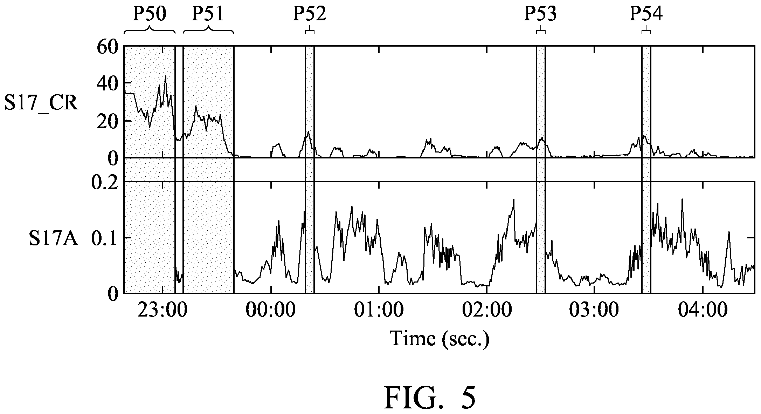

In an embodiment, the turned-on/off state of the switch 14 is also controlled by another control signal S101 generated by the signal-quality analyzer 101. The signal-quality analyzer 101 receives the bio-signal S17 from the bio-sensor 17. In the following embodiment, the bio-signal S17 is a PPG signal for illustration. Referring to FIG. 4, the quality of the bio-signal S17 is low. The signal-quality analyzer 101 takes a time window (such as 30 seconds) each time and extracts beat points (represented by the label "*" in FIG. 4) from the bio-signal S17 in the time window. Then, the signal-quality analyzer 101 calculates the beat intervals between the extracted beat points and calculates the median of the values of the beat intervals (feature values) in the time window and determines each of the values of the beat intervals is in a range from a lower threshold which is equal to, for example, -0.5 times the median (-0.5*median) to an upper threshold which is equal to, for example, 0.5 times the median (0.5*median). When the signal-quality analyzer 101 determines the value of one beat intervals is not in the range (+0.5*median -0.5*median), the signal-quality analyzer 101 replaces the value of the one beat intervals with the median. In other words, when the signal-quality analyzer 101 determines the value of one beat intervals is not in the range (+0.5*median∼-0.5*median), the signal-quality analyzer 101 corrects the value of the one beat intervals with the median. After the determination is finished, the signal-quality analyzer 101 calculates the correction rate which is the percentage of the corrected beat intervals over the total beat intervals. A larger correction rate indicates that the number of corrected beat intervals is larger, so that the quality of the bio-signal S17 is low. The signal-quality analyzer 100 then determines whether each correction rate is larger than a correction threshold. Referring to FIG. 5, a correction-rate signal S17_CR represents the correction rates obtained for several time windows. Referring to FIG. 5, the signal-quality analyzer 100 determines that the correction rates are larger than the correction threshold during period P50∼P55, which indicates that the quality of the bio-signal S17 is low. The switch 14 is turned off according to the control signal S101 during the period P50∼P55. Thus, the bio-signal S17 is not provided to the feature extractor 11 during the period P50∼P55. As shown in FIG. 5, since the feature extractor 11 does not receive any the bio-signal S17 during the period P50∼P55, the feature extractor 11 does not perform the predetermined operation, so that no feature data is obtained during the period P50∼P55. Compared with the feature signal S17A of FIG. 2A which is obtained by the feature extractor 11 through performing the predetermined operation all the time, the number of recodes of feature data in FIG. 5 is less, which indicates that the amount of computation induced by the predetermined operation for the feature extractor 11 is decreased. According to the embodiment, since the feature extractor 11 does not perform the predetermined operation all the time, the power consumption of the physiological monitoring device 1 is reduced..

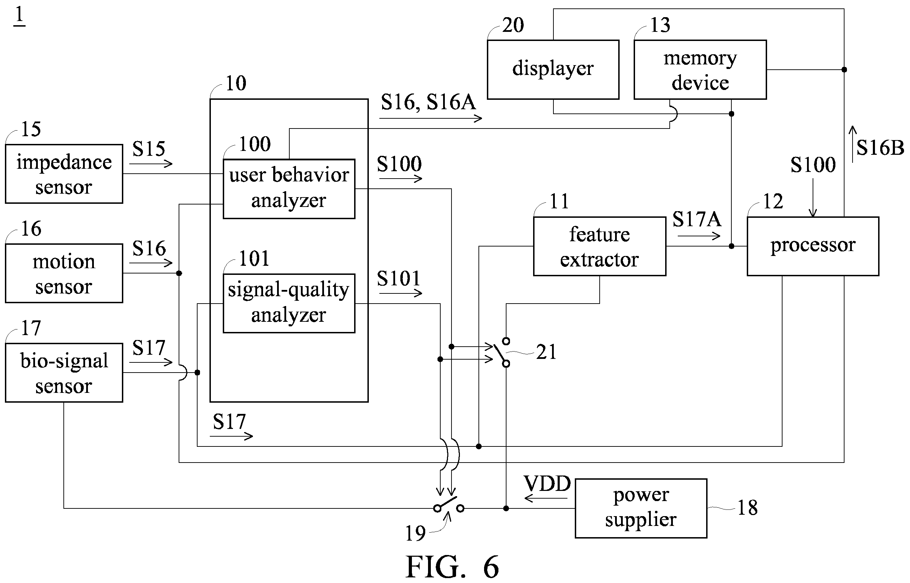

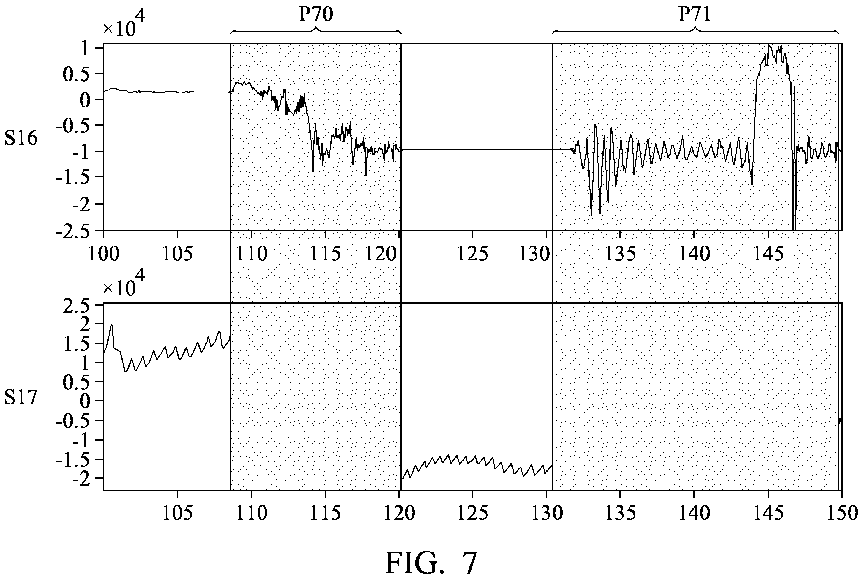

In another embodiment, there is no switch 14, and the transmission path is always provided between the physiological sensor 17 and the feature extractor 11. However, there is a switch 19 coupled to the physiological sensor 17 and the power supplier 18, as shown in FIG. 6. The switch 19 is controlled by the control signals S100 and S101. When the switch 19 is turned on according to the control signal S100 or S101, the power supply 18 provides a supply voltage VDD to power the physiological sensor 17, and then the physiological sensor 17 works to sense a physiological feature of the user. When the switch 19 is turned off according to the control signal S100 or S101, the physiological sensor 17 does not work, and no bio-signal S17 is generated. Referring to FIG. 7, during the periods P70 and P71, there is lager variation on the waveform of the motion signal S16, which indicates that the user is exercising or moving the limbs. Thus, the user behavior analyzer 100 determines that the value of the normalized motion signal S16A is larger than the motion threshold during the periods P70 and P71. Then, the switch 19 is turned off according to the control signal S100 during the periods P70 and P71, so that the supply voltage VDD stops being provided to the physiological sensor 17, and the physiological sensor 17 does not work. Thus, during the periods P70 and P71, the bio-signal S17 is not generated, and the feature extractor 11 does not receives the bio-signal S17. Since the feature extractor 11 does not receive any the bio-signal S17 during the period P70 and P71, the feature extractor 11 does not perform the predetermined operation, so that the amount of computation induced by the predetermined operation for the feature extractor 11 is decreased, thereby reducing the power consumption of the physiological monitoring device 1. Similarly, in another embodiment, the switch 19 may be turned off according to the control signal S101 when the signal-quality analyzer 101 determines that the correction rate is larger the correction threshold. The operations of the user behavior analyzer 100 and the signal-quality analyzer 101 have been descripted above, thus, the related description is omitted. In another embodiment, there is a switch 21 coupled to the feature extractor 11 and the power supplier 18, as shown in FIG. 6. The switch 21 is controlled by the control signals S100 and S101. When the switch 21 is turned on according to the control signal S100 or S101, the power supply 18 provides the supply voltage VDD to power the feature extractor 11, and then the feature extractor 11 works to obtain the feature signal S 17A. In this case, the on/off states of the switches 19 and 21 are controlled by control signal S100 and S101 synchronously. Note that, the power supplier 18 always provides the supply voltage VDD to the impedance sensor 15, the motion sensor 16, and the quality estimator 10.

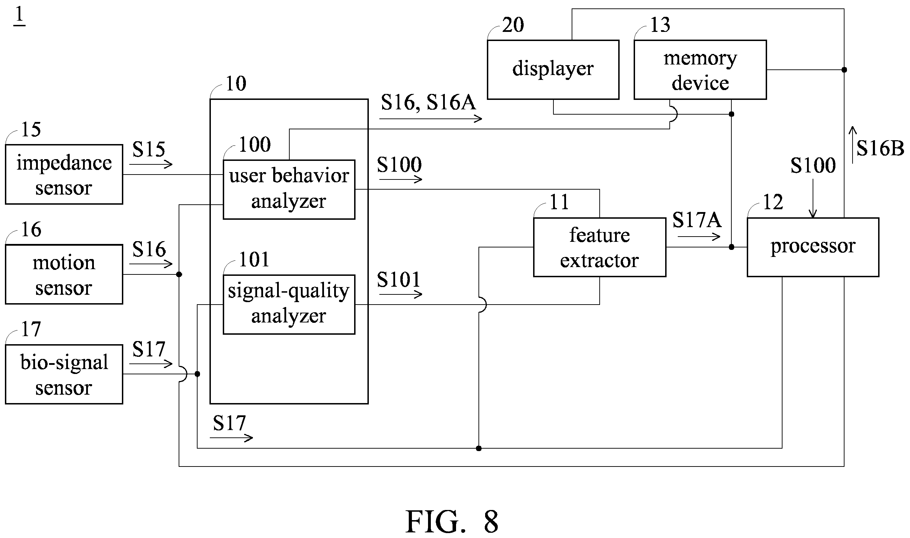

In another embodiment, there is no switch 14, and the transmission path is always provided between the physiological sensor 17 and the feature extractor 11, so that the feature extractor 11 can continuously receive the bio-signal S17. Moreover, there no switch 19 coupled between the physiological sensor 17 and the power supplier 19. Note that, the control signals S100 and S101 are provided to the feature extractor 11, as shown in FIG. 8. When at least one of the user behavior analyzer 100 and the signal-quality analyzer 101 determines that the quality of the bio-signal S 17 is low, the feature extractor 11 is disabled, so that the feature extractor 11 does not performed the predetermined operation on the bio-signal S17, so that the amount of computation induced by the predetermined operation for the feature extractor 11 is decreased, thereby reducing the power consumption of the physiological monitoring device 1. The determination operations of the user behavior analyzer 100 and the signal-quality analyzer 101 have been described above, and the related description is omitted here.

In some embodiment, the power consumption of the physiological monitoring device 1 can be further achieved by decreasing the times of accessing the memory device 13. The memory device 13 may comprise at least one volatile memory (such as an SRAM) and at least one non-volatile memory (such as a flash memory). In the embodiments of FIGs. 1, 6, and 8, the processor 12 receives the motion signal S16 and performs at least one operation on the motion signal S16 to obtain a feature signal S16B. The processor 12 is controlled by the control signal S100. Whether the processor 12 records signal or data in to the memory device 13 is determined by the control signal S100. The normalized motion signal S16A and the feature signal S16B derived from the motion signal S16 can be considered as parameters for determining the motion state. Referring to FIG. 9, the user behavior analyzer 100 determines that the value of the normalized motion signal S16A during the periods P90∼P100 is larger than the motion threshold, which indicates that the user is exercising or moving the limbs during the periods P90-P100. The signals S16A and 16B during the periods P90-P100 are important and helpful for monitoring the motion state of the user. Thus, during the periods P90∼P100, the user behavior analyzer 100 continuously records the normalized motion signal S16A into the volatile memory and the non-volatile memory in the memory device 13, and the processor 12 also continuously records the feature signal S16B into the volatile memory and the non-volatile memory in the memory device 13 according to the control signal S100. However, the motion signal S16 including the X-direction element S16_X, the Y-direction element S16_Y, and the Z-direction element S16_Z is not recorded into the memory device 13 during the periods P90-P100. The user behavior analyzer 100 determines that the value of the normalized motion signal S16A during the static periods (the remaining periods) is not larger than the motion threshold. During each static period, the user behavior analyzer 100 records only the last value of the motion signal S16 into the non-volatile memory. However, the signals 16A and 16B during the static periods are not recorded into the memory device 13. Thus, the motion signal S16, the normalized motion signal S16A, and the feature signal S16B are not continuously recoded into the memory device 13. Since the times of the accessing the memory device 13 is decreased, the power consumption of the physiological monitoring device 1 can be reduced. In an embodiment, the processor 12 can transmit the feature signal S16B to the displayer 20, and then the displayer 20 shows the corresponding values or waveforms related to the feature signal S16B.

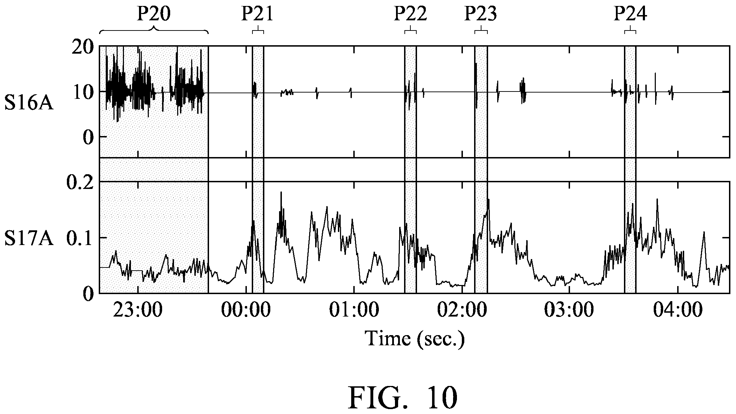

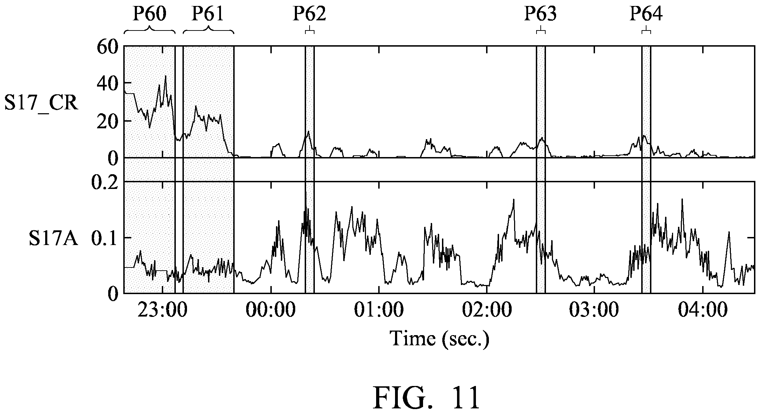

In the above embodiment of FIG. 8, whether the feature extractor 11 performs the predetermined operation on the bio-signal S17 is determined according to the control signals S100 and S101. According to some embodiments, the control signals S100 and S101 are used to control whether the feature exactor 11 records the feature signal S17A into the memory device 13. In these embodiments, the feature extractor 11 continuously receives the bio-signal S17 and also continuously performs the predetermined operation on the bio-signal S 17 to obtain the feature signal S 17A. However, whether the feature exactor 11 records the feature signal S 17A into the memory device 13 is determined by the control signals S100 and S101. For example, referring to FIG. 10, when the user behavior analyzer 100 determines that the value of the normalized motion signal S16A is larger than the motion threshold during the periods P20∼P24, the feature exactor 11 does not record the feature signal S17A into the memory device 13 during the periods P20∼P24 according to the control signal S100 generated by the user behavior analyzer 100. In another example, as shown in FIG. 11, when the bio-signal quality analyzer 101 determines that the correction rates is larger than the correction threshold during the periods P50∼P54, the feature exactor 11 does not record the feature signal S17A into the memory device 13 during the periods P50∼P54 according to the control signal S101 generated by the bio-signal quality analyzer 101. Thus, the feature signal S17A is not continuously recoded into the memory device 13. Since the times of the accessing the memory device 13 is decreased, the power consumption of the physiological monitoring device 1 can be reduced.

In some embodiments, the physiological monitoring device 1 comprises several different physiological sensors 17 to sense at least two of the ECG, PPG, EEG, heart beats, or oxygen saturation of the user. The processor 12 may perform various analyses on the motion signal S16 and the bio-signals S17. The processor 12 performs can analyze the motion signal S16 to determine the user scenario, for example, being sleeping or awake. According to the determined result related to the sleep state, the processor 12 can perform different analysis. For example, when the processor 12 determines that the user is not sleep (that is, the user is awake), the processor 12 performs a basic analysis on the bio-signal S 17 related to the ECG to calculate the heart rate variability (HRV) parameter and determine whether the arrhythmia occurs. When the processor 12 determines that the user is sleeping, the processor 12 not only performs the basic analysis related to the arrhythmia, but also performs advanced analyses on the motion signal S16 and the bio-signals S 17 related to the ECG, PPG, and oxygen saturation to determine the current sleep phase and further determine whether the obstructive sleep apnea (OSA) occurs. As described above, the processor 12 can perform different analyses in the different user scenarios. In detailed, when the user is sleeping, the processor 13 only performs a basic analysis, which consumes less power.

While the invention has been described by way of example and in terms of the preferred embodiments, it is to be understood that the invention is not limited to the disclosed embodiments. On the contrary, it is intended to cover various modifications and similar arrangements (as would be apparent to those skilled in the art). Therefore, the scope of the appended claims should be accorded the broadest interpretation so as to encompass all such modifications and similar arrangements.

## Claims

1. A physiological monitoring device comprising:
a physiological sensor configured to sense a physiological feature to generate a bio-signal;
a quality estimator estimating quality of the bio-signal; and
a feature extractor receiving the bio-signal and performing a predetermined operation to the bio-signal to obtain feature data,
wherein an amount of computation induced by the predetermined operation for the feature extractor is changed according to the estimated quality of the bio-signal.

2. The physiological monitoring device as claimed in claim 1, wherein the estimated quality of the bio-signal is represented by a value, and when the quality estimator determines that the value is larger than a threshold, the amount of computation induced by the predetermined operation is decreased.

3. The physiological monitoring device as claimed in claim 1 or 2,
wherein the estimated quality of the bio-signal is represented by a value, and
wherein when the quality estimator determines that the value is larger than a threshold during a first period, the quality estimator generates a control signal to disable the feature extractor during the first period to decrease the amount of computation induced by the predetermined operation.

4. The physiological monitoring device as claimed in any one of claims 1 to 3, further comprising:
a switch, coupled between the physiological sensor and the feature extractor, receiving the bio-signal,
wherein the estimated quality of the bio-signal is represented by a value, and when the quality estimator determines that the value is larger than a threshold during a first period, the quality estimator generates a control signal to turn off the switch during the first period thereby decreasing the amount of computation induced by the predetermined operation.

5. The physiological monitoring device as claimed in any one of claims 1 to 4,
wherein the estimated quality of the bio-signal is represented by a value, and
wherein when the quality estimator determines that the value is larger than a threshold during a first period, the quality estimator generates a control signal to disable the physiological sensor during the first period thereby decreasing the amount of computation induced by the predetermined operation; and
preferably further comprising:
a power supplier providing a supply voltage for the physiological sensor; and
a switch, coupled between the power supplier and the physiological sensor, receiving the supply voltage,
wherein when the quality estimator determines that the value is larger than the threshold during the first period, the switch is turned off by the control signal during the first period thereby disabling the physiological sensor.

6. The physiological monitoring device as claimed in any one of claims 1 to 5, further comprising
a motion sensor sensing motion of a user and generating a motion signal according to the sensed motion;
wherein the quality estimator receives the motion signal and obtains a value related to the motion signal to represent the estimated quality of the bio-signal,
wherein the quality estimator determines whether the value is larger than a threshold, and when the quality estimator determines that the value is larger than the threshold during a first period, the amount of computation induced by the predetermined operation for the feature extractor is decreased.

7. The physiological monitoring device as claimed in any one of claims 1 to 6, further comprising:
an impedance sensor sensing contact impedance of the physiological sensor and generating an impedance signal according to the sensed contact impedance;
wherein the quality estimator receives the impedance signal and obtains a value of the impedance signal to represent the estimated quality of the bio-signal,
wherein the quality estimator determines whether the value of the impedance signal is larger than a threshold, and when the quality estimator determines that the value of the impedance signal is larger than the threshold during a first period, the amount of computation induced by the predetermined operation for the feature extractor is decreased.

8. The physiological monitoring device as claimed in any one of claims 1 to 7,
wherein the quality estimator receives the bio-signal and extracts feature values from the bio-signal in a time window,
wherein the quality estimator corrects each feature value which is not in a predetermined range and calculates a correction rate for the feature values in the time window to represent the estimated quality of the bio-signal, and
wherein the quality estimator determines whether the correction rate is larger than a threshold, and when the quality estimator determines that the correction rate is larger than the threshold, the amount of computation induced by the predetermined operation for the feature extractor is decreased.

9. A physiological monitoring method comprising:
sensing a physiological feature by a physiological sensor;
estimating quality of the bio-signal;
performing a predetermined operation to the bio-signal to obtain feature data by a feature extractor; and
changing an amount of computation induced by the predetermined operation for the feature extractor according to the estimated quality of the bio-signal.

10. The physiological monitoring method as claimed in claim 9, wherein the step of estimating quality of the bio-signal comprises:
obtaining a value to represent the estimated quality of the bio-signal;
determining whether the value is larger than a threshold,
wherein when it is determined that the value is larger than the threshold, the amount of computation induced by the predetermined operation is decreased, and/or
wherein the step of changing the amount of computation induced by the predetermined operation for the feature extractor comprises:
when it is determined that the value is larger than the threshold during a first period, disabling the feature extractor during the first period to decrease the amount of computation induced by the predetermined operation, and/or
when it is determined that the value is larger than the threshold during a first period, disabling the physiological sensor, preferably by stopping providing a supply voltage to the physiological sensor, during the first period thereby decreasing the amount of computation induced by the predetermined operation.

11. The physiological monitoring method as claimed in claim 9 or 10, further comprising:
providing a transmission path between the physiological sensor and the feature extractor,
wherein the step of estimating quality of the bio-signal comprises:
obtaining a value to represent the estimated quality of the bio-signal; and
determining whether the value is larger than a threshold, and
wherein the step of changing the amount of computation induced by the predetermined operation for the feature extractor comprises:
when it is determined determines that the value is larger than the threshold during a first period, cutting off the transmission path during the first period thereby decreasing the amount of computation induced by the predetermined operation.

12. The physiological monitoring method as claimed in any one of claims 9 to 11, further comprising
sensing motion of a user to generate a motion signal;
wherein the step of estimating quality of the bio-signal comprises:
obtaining a value related to the motion signal to represent the estimated quality of the bio-signal; and
determining whether the value is larger than a threshold, and
wherein the step of changing the amount of computation induced by the predetermined operation for the feature extractor comprises:
when it is determined that the value is larger than the threshold during a first period, decreasing the amount of computation induced by the predetermined operation.

13. The physiological monitoring method as claimed in any one of claims 9 to 12, further comprising:
sensing contact impedance of the physiological sensor to generate an impedance signal;
wherein the step of estimating quality of the bio-signal comprises:
obtaining a value of the impedance signal to represent the estimated quality of the bio-signal; and
determining whether the value is larger than a threshold, and
wherein the step of changing the amount of computation induced by the predetermined operation for the feature extractor comprises:
when it is determined that the value of the impedance signal is larger than the threshold during a first period, decreasing the amount of computation induced by the predetermined operation.

14. The physiological monitoring method as claimed in any one of claims 9 to 13,
wherein the step of estimating quality of the bio-signal comprises:
extracting feature values from the bio-signal in a time window;
correcting each feature value which is not in a predetermined range;
calculating a correction rate for the feature values in the time window to represent the estimated quality of the bio-signal, and
determining whether the value is larger than a threshold, and
wherein the step of changing the amount of computation induced by the predetermined operation for the feature extractor comprises:
when it is determined that the correction rate is larger than the threshold, decreasing the amount of computation induced by the predetermined operation.

15. The physiological monitoring device as claimed in any one of claims 1 to 8, or the physiological monitoring method as claimed in any one of claims 9 to 14, wherein the bio-signal is an electrocardiography (ECG) signal, an electroencephalograph (EEG) signal, an electromyography (EMG) signal, an electrooculography (EOG) signal, an electroretinogram (ERG) signal, an electrogastrography (EGG) signal, an electroneurogram (ENG), a photoplethysmogram (PPG) signal, or a heart-beat signal.
